(19) Europäisches Patentamt / European Patent Office / Office européen des brevets

(11) **EP 3 647 428 A1**

(12) # EUROPEAN PATENT APPLICATION
published in accordance with Art. 153(4) EPC

(43) Date of publication:
06.05.2020 Bulletin 2020/19

(51) Int Cl.:
*C12P 1/00* (2006.01)          *B01D 61/14* (2006.01)
*C12M 1/12* (2006.01)          *C12P 7/56* (2006.01)

(21) Application number: 18824450.3

(22) Date of filing: 29.06.2018

(86) International application number:
**PCT/JP2018/024978**

(87) International publication number:
**WO 2019/004478 (03.01.2019 Gazette 2019/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 30.06.2017 JP 2017128606

(71) Applicant: **Toray Industries, Inc.**
**Tokyo 103-8666 (JP)**

(72) Inventors:
• **SHIMURA Fumi**
  **Otsu-shi**
  **Shiga 520-8558 (JP)**
• **KANAMORI Satoko**
  **Otsu-shi**
  **Shiga 520-8558 (JP)**
• **TAKAGI Ryota**
  **Otsu-shi**
  **Shiga 520-8558 (JP)**

(74) Representative: **Hoefer & Partner Patentanwälte mbB**
**Pilgersheimer Straße 20**
**81543 München (DE)**

(54) **PRODUCTION METHOD AND PRODUCTION DEVICE FOR CHEMICAL PRODUCT BY CONTINUOUS FERMENTATION**

(57)     Provided is a chemical product production method wherewith filterability and productivity with respect to a target substance can be both achieved. The present invention is provided with a mechanism for removing solid contents in a pipe connecting a fermentation tank and a filter separation module, and adds the solid contents removed by the removal mechanism to a culture liquid.

[Fig. 1]

EP 3 647 428 A1

**Description**

TECHNICAL FIELD

The present invention relates to a method and apparatus for producing a chemical through continuous fermentation.

BACKGROUND ART

The continuous fermentation method is one of substance production methods involving cell culture (hereinafter, the "cell" encompasses a "microorganism").

In the continuous fermentation method, a fresh medium is supplied to a fermentor and the same amount of culture liquid is discharged to the outside of the tank, and the liquid volume in the fermentor can thereby be always kept constant. In the continuous fermentation method, in order to maintain the microorganism concentration in the fermentor, it has been proposed to separate microorganisms from the discharged culture liquid with a separation membrane and the filtrate from which the microorganism have been removed is used for the purification of a target product while refluxing the separated microorganisms to the culture liquid (see, Patent Literature 1).

Meanwhile, in the continuous fermentation method, flow channels and pores of the membrane are sometimes clogged with solid matter in the culture liquid during the filtration operation, as a result, the filtration capacity may be reduced or supply of the culture liquid to the membrane module may become impossible.

Patent Literature 2 discloses a method of removing large solid matter in advance by a removal mechanism such as screen to remove solid matter entangled with a reactor in the methane fermentation.

CITATION LIST

PATENT LITERATURE

Patent Literature 1: WO 07/097260
Patent Literature 2: JP-A-2006-326534

SUMMARY OF INVENTION

TECHNICAL PROBLEM

An object of the present invention is to provide a method for producing a chemical, in which both the productivity of a target substance and the filterability can be satisfied.

SOLUTION TO PROBLEM

The present invention provides the followings.

[1] An apparatus for producing a chemical, including:

a fermentor for housing a feedstock and a culture liquid containing a microorganism capable of producing methane through fermentation from the feedstock,
an external pressure-type membrane separation module for separating the microorganism from the culture liquid to obtain a filtrate and a concentrated liquid,
a supply piping for supplying the culture liquid in the fermentor to the membrane separation module,
a reflux piping for refluxing the concentrated liquid from the membrane separation module into the fermentor, and
a solid matter removal mechanism disposed on the supply piping for trapping part of the solid matter in the culture liquid, wherein
the membrane separation module includes a container, a separation membrane housed in the container, a first opening allowing the culture liquid flowing into the container to pass therethrough, and a second opening allowing the concentrated liquid flowing out of the container to pass therethrough, the openings being provided in the container,
the first opening and the second opening are arranged apart from each other in the longitudinal direction of the container,
the first opening and/or the second opening is provided on a side surface of the container, and
an aperture size of the solid matter removal mechanism is 1/3 or less of a smaller one of major axes of the first and second openings provided on the side surface of the container.

[2] The apparatus for producing a chemical according to [1], wherein the aperture size of the solid matter removal mechanism is 0.1 mm or more.

[3] The apparatus for producing a chemical according to [1] or [2], wherein the aperture size of the solid matter removal mechanism is 10 mm or less.

[4] The apparatus for producing a chemical according to [3], wherein the aperture size of the solid matter removal mechanism is 2 mm or less.

[5] The apparatus for producing a chemical according to any one of [1] to [4], further including a solid matter addition piping through which the solid matter trapped by the solid matter removal mechanism is added to the culture liquid in the fermentor.

[6] A method for producing a chemical by using the apparatus for producing a chemical according to any one of [1] to [5], including:
a step of flowing a culture liquid into a membrane separation module such that the membrane surface linear velocity $v$ [$m^3/m^2/s$] of the culture liquid in the membrane separation module in the longitudinal direction of the container and the smaller one A [m] of major axes of the first and second openings provided on the side surface of the container satisfy $v>0.8(2gA(S-1))^{0.5}$.

[7] A method for producing a chemical by using the apparatus for producing a chemical according to any one of [1] to [5], including:
a step of directly adding the solid matter trapped by the solid matter removal mechanism to the culture liquid in the fermentor.

[8] A method for producing a chemical by using the apparatus for producing a chemical according to any one of [1] to [5], including:
a step of adding the solid matter trapped by the solid matter removal mechanism to the culture liquid in the fermentor through the reflux piping.

[9] A method for producing a chemical by using the apparatus for producing a chemical according to any one of [1] to [5], which further includes a feedstock supply piping connected to the fermentor, including:

a step of adding a fermentation feedstock to the fermentor through the feedstock supply piping, and
a step of adding the solid matter trapped by the solid matter removal mechanism to the culture liquid in the fermentor through the feedstock supply piping.

ADVANTAGEOUS EFFECTS OF INVENTION

Clogging due to solid matter is prevented from occurring in a membrane separation module by providing a solid matter removal mechanism between a fermentor and the membrane separation module and at the same time, setting the aperture size of the mechanism relative to the major axis of an opening at the location where the flow channel of the culture liquid in the membrane separation module is bent.

In the solid matter removal mechanism, if the aperture size is excessively reduced, clogging of the mechanism itself which removing solid matter frequently occurs. On the other hand, if the aperture size is increased, solid matter flows into the membrane separation module to cause clogging of the flow channel. In the present invention, the aperture size is appropriately set, and stable operation can thereby be realized while preventing both the solid matter removing mechanism and the membrane separation module from clogging.

BRIEF DESCRIPTION OF DRAWINGS

[Fig. 1] A schematic diagram illustrating the membrane separation continuous fermentation apparatus as one embodiment of the present invention.
[Fig. 2] A cross-sectional view illustrating one example of the external pressure-type membrane separation module.
[Fig. 3] A cross-sectional view illustrating another example of the external pressure-type membrane separation module.
[Fig. 4] A schematic diagram illustrating the membrane separation continuous fermentation apparatus in Reference Example.

DESCRIPTION OF EMBODIMENTS

1. Continuous Fermentation Apparatus

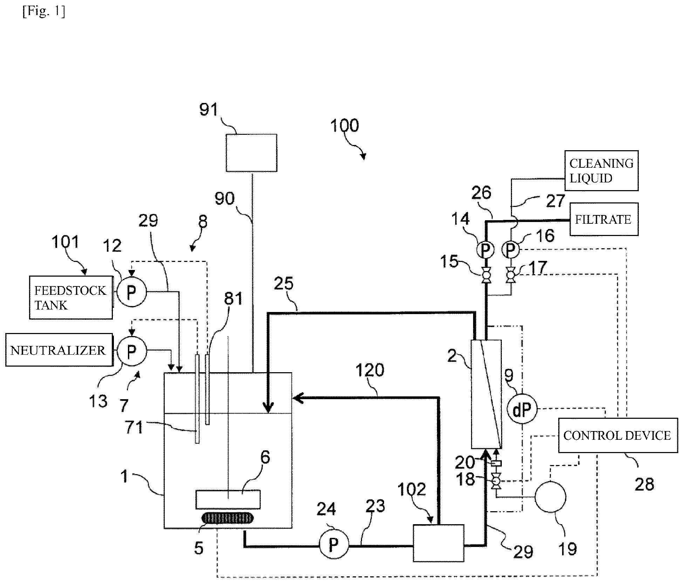

One example of the continuous fermentation apparatus is described by referring to Fig. 1. Fig. 1 is a schematic side elevation of the continuous fermentation apparatus of this embodiment.

As illustrated in Fig.1, the continuous fermentation apparatus 100 includes a fermentor 1, a membrane separation module 2, and a piping connecting the fermentor 1 to the membrane separation module 2. The fermentor 1 and the membrane separation module 2 are connected to form a circulation system.

1-1. Fermentor

The fermentor 1 is configured to house a culture liquid in the inside thereof. The culture liquid is described later.

Specifically, the fermentor 1 is made of a material excellent in pressure resistance and antifouling property. The fermentor 1 may have various shapes such as cylindrical shape and polygonal columnar shape. When the fermentor 1 has the cylindrical shape, stirring efficiency of a culture liquid is good.

The fermentor 1 is configured, if desired, to allow for sterilization and hermetic sealing. Substances such as fermentation feedstock housed in the fermentor 1 are stirred by the later-described stirring device 6. From the viewpoint of stirring efficiency of a culture liquid, the fermentor 1 has preferably the cylindrical shape.

1-2. Membrane Separation Module

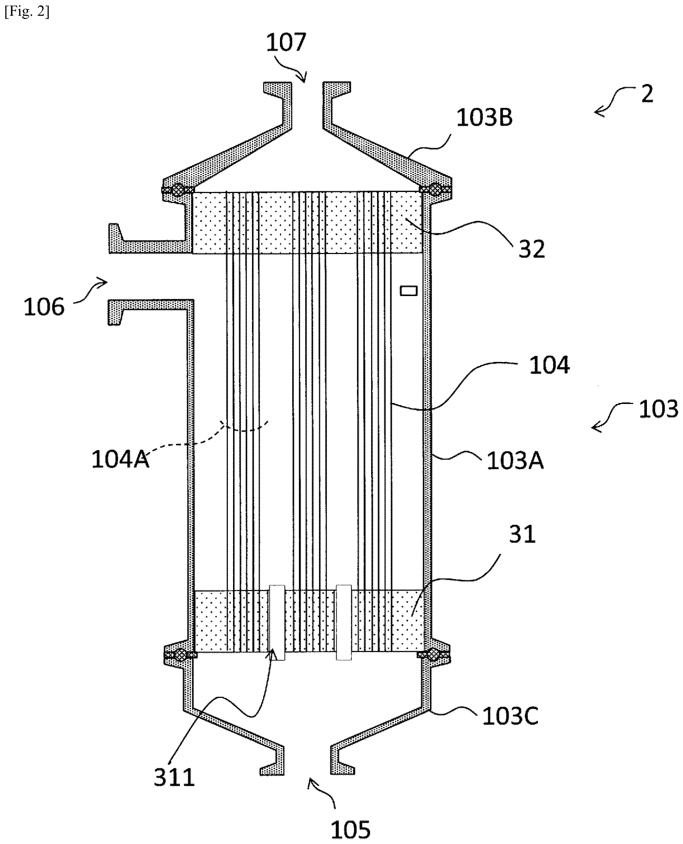

The membrane separation module 2 is not limited to a specific configuration and may be sufficient if it can separate microorganisms from the culture liquid and it enables to obtain a concentrated liquid and a filtrate can be obtained by filtering the culture liquid. As illustrated in Fig. 2, the membrane separation module 2 includes a container 103 and a number of separation membranes 104 housed in the container.

In this embodiment, the separation membrane 104 is a hollow fiber membrane. In a module having a hollow fiber membrane, the membrane area per volume of the module can be made larger than in a module having a flat sheet membrane.

In addition, the separation membrane module 2 includes a plurality of separation membrane bundles 104A. The separation membrane bundle 104A contains a plurality of separation membranes 104.

The separation membrane may be either an organic membrane or an inorganic membrane. Any separation membrane may be used as far as it can be used for removal of microorganisms from a culture liquid. The separation membrane includes, for example, membranes made of polyvinylidene fluoride, polysulfone, polyethersulfone, polytetrafluoroethylene, polyethylene, polypropylene, and ceramics. Among these, a polyvinylidene fluoride-made separation membrane is preferred because it is resistant to fouling by a fermentation liquid, easy to clean and furthermore, excellent in durability against cleaning with a gas.

In order to effectively separate microorganisms in the fermentation liquid, the separation membrane is preferably a porous membrane having pores with an average pore diameter of 0.001 $\mu$m or more and less than 10 $\mu$m.

The average pore diameter of the membrane is determined according to the method described in ASTM: F316-86 (another name: half dry method). The half dry method determines the average pore diameter of a minimum pore diameter layer of a membrane.

The standard measurement conditions in measurement of an average pore diameter by the half dry method are as follows.

Liquid used: ethanol

Measurement temperature: 25°C

Pressure rise rate: 1 kPa/sec

The average pore diameter [$\mu$m] is determined according to the following equation:

$$\text{Average pore diameter } [\mu m] = (2860 \times \text{surface tension } [mN/m])/\text{half dry air pressure } [Pa]$$

The surface tension at 25°C of ethanol is 21.97 mN/m (The Chemical Society of Japan, *Kagaku Binran Kisohen Kaitei 3rd Edition*, pp. II-82, Maruzen, 1984) and therefore, under the standard measurement conditions of the present invention, the average pore diameter can be determined according to the following equation:

4

$$\text{Average pore diameter [µm]} = 62834.2/(\text{half dry air pressure [Pa]})$$

The membrane separation module 2 is preferably an external pressure type. In the external pressure-type membrane separation module, the culture liquid flows on the outer side of a hollow fiber membrane, and the filtrate flows on the inner side (i.e., hollow part) of the hollow fiber membrane. In other words, at the time of filtration, a liquid flows from the outer side toward the inner side of a hollow fiber membrane. A concentrated liquid containing substances, which is not filtered, flows on the outer side of the hollow fiber membrane. In the internal pressure type, the direction of the liquid flow during filtration is reversed.

The outer diameter of the external pressure-type hollow fiber membrane is preferably 0.5 mm or more and 3 mm or less. When the outer diameter is 0.5 mm or more, the resistance of the filtrate flowing through the hollow fiber membrane can be reduced to a relatively low level. In addition, when the outer diameter is 3 mm or less, the hollow fiber membrane can be prevented from collapsing due to an outer pressure from a fermentation liquid or gas.

The inner diameter of an inner pressure-type hollow fiber membrane is preferably 0.5 mm or more and 3 mm or less. When the inner diameter is 0.5 mm or more, the resistance of a fermentation liquid flowing through the hollow fiber membrane can be reduced to a relatively low level. In addition, when the inner diameter is 3 mm or less, a membrane surface area can be ensured and therefore, an increase in size of the membrane separation module can be suppressed.

The container 103 of the membrane separation module is made of a material having excellent pressure resistance and may have any shape as long as it enables to supply a fermentation liquid to the primary side of the membrane, such as cylindrical shape and polygonal columnar shape. In consideration of the flow of fermentation liquid and the handling property, the container has preferably a cylindrical shape. In addition, the membrane separation module may be an immersion-type membrane separation device in which a separation membrane is immersed in a water tank.

In Fig. 2, the container 103 of the membrane separation module has a cylindrical shape, and a hollow fiber membrane is disposed such that its longitudinal direction extends along the height direction of the container 103 (longitudinal direction of the container).

In this embodiment, the container 103 includes a container body 103A, an upper cap 103B, and a lower cap 103C.

In this embodiment, a bottom opening 105 is provided at the bottom of the lower cap 103C, and a side opening 106 is provided on a side surface of the container body 103A.

The culture liquid flows into the container 103 through the bottom opening 105 and is supplied between separation membranes 104. The culture liquid is separated into a concentrated liquid and a filtrate by the separation membranes 104. The concentrated liquid passes through the separation membrane 104 (between separation membranes 104) and is discharged to the outside of the container 103 through the side opening 106. The concentrated liquid is returned to the fermentor 1 as described later. The filtrate passes through inside the separation membrane 104 and is discharged to the outside of the container 103 through the upper opening 107 provided in the upper cap 103B.

The container of the separation membrane module includes a first opening and a second opening. The first opening and the second opening are arranged apart from each other in the longitudinal direction of the container. The first opening and/or the second opening is provided parallel to the longitudinal direction of the container (i.e., the longitudinal direction of the hollow fiber membrane). More specifically, the culture liquid flowed into the container passes through the first opening, and the concentrated liquid flowing out to the outside of the container passes through the second opening. Furthermore, the flow direction of the culture liquid flowing into the container through the first opening and/or the flow direction of the concentrated liquid flowing out to the outside of the container though the second opening is perpendicular to the longitudinal direction of the container (i.e., the longitudinal direction of the hollow fiber membrane).

Here, in the container 103, the bottom opening 105 corresponds to the first opening, and the side opening 106 corresponds to the second opening. That is, in this embodiment, the second opening is provided on a side surface of the container. The present invention is not limited thereto, and only the first opening or both the first and second openings may be provided on a side surface of the container. For example, instead of the bottom opening 105, the first opening may be provided on a side surface of the container body 103A or lower cap 103C.

The first opening and the second opening are arranged apart from each other in the longitudinal direction of the container and moreover, hollow fiber membranes are housed parallel to the longitudinal direction of the container, as a result, the culture liquid and the concentrated liquid flow from the first opening toward the second opening along the longitudinal direction of the container, i.e., along the longitudinal direction of the hollow fiber membrane.

Furthermore, the first opening and/or the second opening is provided on a side surface in the longitudinal direction of the container, and this allows formation of a bent part in the flow channel through which the culture liquid flowing into the membrane separation module or the concentrated liquid flows. Here, the bent part means a portion where the flow of the culture liquid is changed at a right angle or an angle close to it. In the case where one of the first opening and the second opening is provided on a side surface in the longitudinal direction of the container, one bent part is formed, and in the case where both openings are provided on a side surface in the longitudinal direction of the container, two bent parts are formed.

Because the flow channel has the bent part, an opening for collecting a filtered liquid having passed through the hollow part of the hollow fiber membrane can be provided, or a drain opening for discharging a concentrate accumulated inside the container can be provided, in a transverse direction, i.e., radial direction of the hollow fiber membrane housed in the container. As described later, solid matter in the culture liquid or concentrated liquid readily stays or deposits in the bent part and therefore, the relationship between the major axis of the opening and the size of the solid matter affects how easily clogging by solid matter occurs in the membrane separation module.

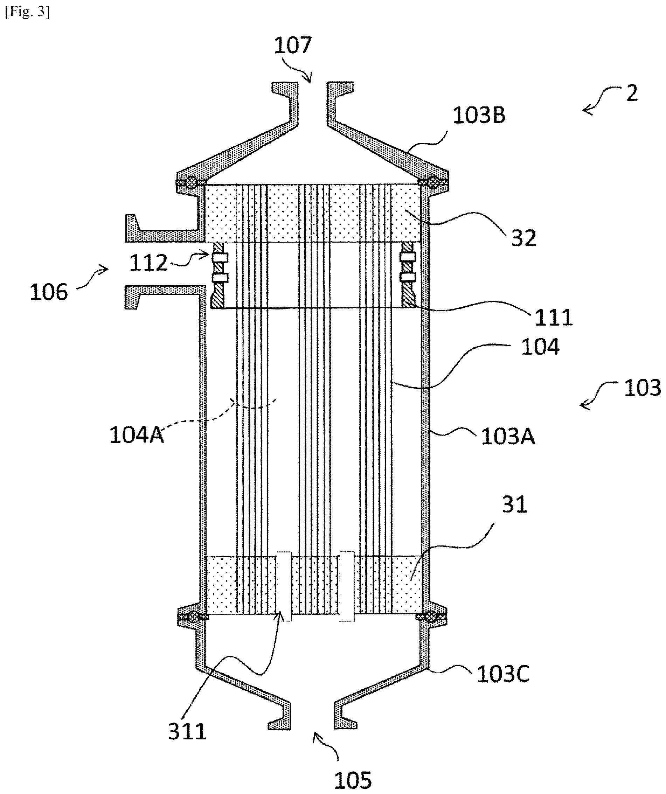

In the embodiment illustrated in Fig. 3, the membrane separation module 2 includes a flow-regulating member 111, in addition to the configuration of Fig. 2. The flow-regulating member 111 is a member for regulating a liquid flow in the membrane separation module. In this embodiment, the flow-regulating member 111 is a cylindrical member and is disposed near the upper end of the container body 103A inside the container 103. The flow-regulating member 111 has a plurality of flow-regulating holes 112 on a side surface (a surface parallel to the side surface of the container body 103A). The flow-regulating member 111 is disposed such that the side opening 106 overlaps with the flow-regulating hole 112.

In this embodiment, a concentrated liquid passes through the separation membrane 104 (between separation membranes 104), gets through the flow-regulating holes 112 of the flow-regulating member 111 and is further discharged to the outside of the container 103 through the side opening 106.

More specifically, as in this embodiment, when the membrane separation module has a flow-regulating member 111 disposed inside the container, the concentrated liquid passes through both the flow-regulating hole 112 and the side opening 106. Accordingly, a smaller one of the diameter of the flow-regulating hole 112 and the diameter of the side opening 106 substantially defines the diameter of the second opening. In other words, a smaller one of the major axis of the flow-regulating hole 112 and the major axis of the side opening 106 is the major axis of the second opening. When the membrane separation module includes a flow-regulating member, the flow-regulating hole of the flow-regulating member is, in many cases, smaller than the opening of the container, which is on the outer side relative to the hole, and therefore, the major axis of the flow-regulating hole is regarded as the major axis of the second opening. That is, in this embodiment, the major axis of the flow-regulating hole 112 is regarded as the major axis of the second opening.

1-3. Circulation System

The continuous fermentation apparatus 100 includes a circulation system for circulating a culture liquid between the fermentor 1 and the membrane separation module 2. Specifically, the continuous fermentation apparatus 100 includes a supply piping 23 for allowing the fermentor 1 to communicate with the primary side of the membrane separation module 2, and a reflux piping 25 for returning a concentrated liquid, which did not permeate the separation membrane of the membrane separation module 2, to the fermentor 1. The piping 23 may be connected to either the lower part or upper part of the membrane separation module 2. In addition, on the piping 23 for supplying a culture liquid from the fermentor 1 to the membrane separation module 2, a circulation pump 24 is disposed. The circulation pump 24 operates to feed a culture liquid from the fermentor 1 toward the membrane separation module 2.

1-4. Solid Matter Removal Mechanism

The continuous fermentation apparatus 100 has a solid matter removal mechanism 102 in the middle of the piping 23, which allows the fermentor 1 to communicate with the membrane separation module 2 and supplies a culture liquid from the fermentor 1 to the membrane separation module 2.

The solid matter removal mechanism 102 traps solid matter larger than microorganisms that the membrane separation module 2 can separate, and part of the solid matter can thereby be removed from a culture liquid.

The solid matter removal mechanism specifically includes a filter medium. The filter medium is a so-called net-like, slit-like or porous filter. The filter medium is, for example, a strainer, a sintered filter, a screen filter, a drum filer, a disc filter, a mesh filter, or a bar screen and may be sufficient as long as it can separate coarse solid matter present in the water flow, and a filter medium usable in a closed system is preferred.

The size of the solid matter separated by the solid material removal mechanism is determined by the aperture size of the filter medium. The aperture size is a minor axis of the opening that the filter medium has. When the filter medium is a mesh formed from fibers, the gap between fibers is the opening. When a filter medium has a slit, the slit is the opening. When the filter medium is porous, the pore is the opening.

The aperture size of the solid matter removal mechanism is preferably 1/3 or less of a smaller one of the major axis of the first opening and the major axis of the second opening which are provided on a side surface of the container of the membrane separation module. The aperture size is more preferably 1/4 or less or 1/5 or less of a smaller one of major axes of the openings above. The aperture size is preferably 0.1 mm or more while satisfying the condition of being 1/3 of the major axis of the first opening or second opening. In addition, the aperture size is preferably 10 mm or less, more preferably 2 mm or less.

As described above, a bent part is formed in the flow channel through which the culture liquid flowing into the membrane separation module or the concentrated liquid flows. The flux of a liquid decreases in the bent part or in the vicinity thereof, allowing solid matter to readily stay. The aperture size is 1/3 or less of a smaller one of major axes of the first and second openings, so that the deposition of solid matter in the flow channel of the membrane separation module 2 can be prevented. The reason why the basis is the major axis but not the minor axis of the first or second opening is considered to be that even if the solid matter is larger than the minor axis, though not trapped, when the size thereof is 1/3 or less of the major axis, the solid matter rotates and thereby can pass through the first and second openings.

In addition, the aperture size is 0.1 mm or more, so that clogging of the solid matter removal mechanism itself can be prevented and the discharge frequency of the trapped solid matter can be decreased.

In the embodiment illustrated in Fig. 2, only a side opening 106 is provided on a side surface of the container and therefore, the aperture size may be 1/3 or less of the major axis of the side opening 106. In the case where a first opening is present on a side surface of the container body 103A or lower cap 103C, instead of the bottom opening 105, the aperture size should only be 1/3 or less of a smaller one of the major axis of the first opening and the major axis of the side opening 106 serving as the second opening.

In the embodiment illustrated in Fig. 3, only a side opening 106 is provided on a side surface of the container and since a flow-regulating member 111 is disposed inside the container and a concentrated liquid passes through the flow-regulating hole 112 of the flow-regulating member and the side opening 106, the aperture size may be 1/3 or less of the major axis of the flow-regulating hole 112.

In the case where placements of the side opening 106 and the flow-regulating member 111 are the same as in Fig. 3 and a first opening is present on a side surface of the container body 103A or lower cap 103C, instead of the bottom opening 105, while the location of the side opening 106, a smaller one of the major axis of the flow-regulating hole 112 and the major axis of the first opening is the basis for determining the aperture size of the solid matter removal mechanism.

In the case where the membrane separation module is tubular and includes a flow-regulating member having a side-surface flow-regulating hole, the second opening is usually provided on a side surface of the container. In this case, the flow-regulating hole is preferably overlapped with the second opening but may not be overlapped. On the other hand, in the case where the membrane separation module does not include a flow-regulating member, the second opening may be provided at the end of the container (a portion except for the side surface). When the second opening is provided at the end of the container and the first opening is provided on a side surface of the container, the major axis of the first opening serves as the basis for the aperture size of the solid matter removal mechanism.

Only one solid matter removal mechanism may be provided, or a standby system may be provided as a backup during the discharging process of solid matter.

1-5. Solid Matter Addition Mechanism

In the example of Fig. 1, the continuous fermentation apparatus 100 includes a solid matter addition piping 120 directly connecting the solid matter removal mechanism 102 and the fermentor 1.

The solid matter addition piping 120 is one example of the solid matter addition mechanism for adding solid matter trapped by the solid matter removal mechanism 102 to a culture liquid in the fermentor 1. The solid matter addition mechanism may include a device such as pump (not shown). When the solid matter once removed is added to a culture liquid, soluble substances adsorbed to the solid matter can also be returned to the culture liquid, so that reduction in the concentration of a substance serving as a substrate of fermentation can be prevented and the productivity of the target substance can be maintained high.

The solid matter addition mechanism is sufficient if solid matter can be eventually added to a culture liquid in the fermentor, and besides direct addition to the fermentor, as long as it is applied to the configuration of Fig. 1, the mechanism may be configured to add the solid matter to a culture liquid in the fermentor 1 through the reflux piping 25, or the solid matter may be added to the fermentor 1 though a piping for supplying the feedstock from a feedstock tank 101.

1-6. Other Mechanisms

The continuous fermentation apparatus 100 includes a control device 28. The control device 28 can perform various calculations. In addition, the control device 28 controls operation of each unit in the continuous fermentation apparatus 100 based on the detection results of various sensors, the inputs from users, and various settings.

In this embodiment, in addition to the above-described configuration, the continuous fermentation apparatus 100 further includes a temperature control unit 5, a stirring device 6, a pH control unit 7, a level control unit 8, a gas tank 91, etc.

The temperature control unit 5 includes a temperature sensor and a temperature regulating unit. The temperature sensor detects the temperature of a culture liquid in the fermentor 1. Under the control by the control device 28, the temperature regulating unit works so that the detection results of the temperature sensor fall within a predetermined range. Consequently, the temperature in the fermentor 1 is kept constant, and a temperature environment suited for fermentation or microorganism proliferation is thereby maintained. The temperature regulating unit can have one or both of heating and cooling functions.

The stirring device 6 keeps an appropriate fermentation environment by stirring a culture liquid in the fermentor 1.

The pH control unit 7 includes a pH sensor 71 and a neutralizer supply pump 13. The pH sensor 71 detects the pH of a culture liquid in the fermentor 1. The neutralizer supply pump 13 is disposed on a piping connecting a neutralizer tank and the fermentor 1 and adds a neutralizer into the fermentor 1. The neutralizer supply pump 13 works based on the control of the control device 28 so that the detection results of the pH sensor 71 fall within a predetermined range. An acid or an alkali is used as the neutralizer.

The level control unit 8 includes a level sensor 81 and a feedstock supply pump 12. The feedstock supply pump 12 is disposed on a piping connecting the feedstock tank 101 and the fermentor 1. Based on the control of the control device 28, the feedstock supply pump 12 operates to supply a medium to the fermentor 1 when the detection results of the level sensor 81 show that the liquid level of a culture liquid in the fermentor 1 falls below a predetermined lower limit, and the operation of the feedstock supply pump 12 is stopped when the detection results show that the liquid surface reaches the upper limit. In this way, the amount of a culture liquid in the fermentor 1 is kept appropriate.

The gas tank 91 is connected to the fermentor 1 via a discharge line 90. The gas produced in the fermentor 1 is discharged through the discharge line 90 and stored in the gas tank 91.

Furthermore, the continuous fermentation apparatus 100 includes a piping 26 that is connected to the membrane separation module 2 and discharges a filtrate (i.e., permeated liquid) to the outside of the apparatus. A filtration pump 14 is provided on the piping 26 and at the same time, a filtration valve 15 is provided between the filtration pump and the membrane separation module 2.

The continuous fermentation apparatus 100 includes a configuration for backwashing of the membrane separation module 2. The backwashing is to clean a separation membrane by letting a liquid for cleaning (hereinafter, sometimes referred to as "cleaning liquid") pass through the separation membrane from the secondary side to primary side thereof. The continuous fermentation apparatus 100 includes a cleaning liquid tank for housing a cleaning liquid, a cleaning piping 27 connecting the cleaning liquid tank and the secondary side of the membrane separation module 2, a cleaning pump 16 provided on the cleaning piping 27, and a cleaning valve 17 provided between the cleaning pump 16 and the membrane separation module 2. A cleaning liquid is fed toward the membrane separation module 2 by the cleaning pump 16. The cleaning liquid may be a filtrate having permeated the separation membrane or water supplied from outside the system. In addition, the water may contain any chemical liquid.

The cleaning piping 27 may have a pressure gauge, a flow meter, etc.

A pressure difference control unit 9 can detect a transmembrane pressure difference (TPD) of the membrane separation module 2. In other words, the unit detects a pressure difference between the primary side (the side to which a culture liquid is supplied) and secondary side (the side from which a filtrate is discharged) of the membrane separation module 2. The transmembrane pressure difference works out to a driving force of filtration.

Furthermore, the continuous fermentation apparatus 100 includes a configuration involved in scrubbing. The scrubbing is a cleaning method in which a gas is supplied to the primary side of the membrane separation module 2 and by making use of oscillation of a liquid and the gas, which occurs during passing of the gas through the membrane separation module 2, substances attached to the surface of the separation membrane are removed from the surface of the separation membrane.

In the continuous fermentation apparatus 100, among others, a gas is supplied to the membrane separation module 2 from the lower part of the membrane separation module 2 and/or the piping 23 communicating between the fermentor 1 and the membrane separation module 2. In particular, as the configuration involved in the scrubbing, a gas supply device for the membrane separation module, a gas supply port, and a mechanism capable of regulating a supply rate of a gas from a gas supply source is provided. A gas supplied or a gas produced by metabolism of microorganisms may be recovered and again supplied into the membrane separation module 2.

Specifically, the continuous fermentation apparatus 100 includes a gas supply control valve 18 and a gas supply device 19.

The gas supply device 19 is connected, with respect to the membrane separation module 2, to the primary side of the separation membrane module 2, that is, to the side to which a culture liquid is supplied, via a piping.

The gas supply port is a portion from which a gas is released into a culture liquid. The gas supply port is preferably formed to permit generation of bubbles capable of cleaning the membrane surface therewith. The bubbles generated may be either a fine bubble or a coarse bubble. The size of the bubble is changed by changing the shape of the gas supply port according to conditions, such as the kind of the separation membrane and a gas diffusion amount. The gas supply port may be formed by providing an air discharge hole in a piping made of polyvinyl chloride or stainless steel, and it is also possible to use a diffuser tube, etc. using a porous rubber, ceramic or membrane. The size of the gas supply port may be any size as long as it can supply a specified amount of a gas and at the same time, is large enough to cause no clogging by a fermentation liquid.

In this way, in Fig. 1, the gas supply port may be provided in the lower part of the membrane separation module 2, or in the configuration of supplying a culture liquid from the fermentor to the membrane separation module 2 by using a pump, the gas supply port may be provided either between the fermentor and the pump or between the pump and the membrane separation module 2.

2. Method for Producing Chemicals

The production method of this embodiment is a method for producing a chemical through continuous fermentation. The chemical is methane and the method includes the following steps (a) to (e):

(a) a fermentation step of fermenting an microorganism in a culture liquid within a fermentor to produce methane from a feedstock,
(b) a filtration step of filtering a culture liquid having passed through the fermentation step with the membrane separation module to obtain a filtrate and a concentrated liquid,
(c) a reflux step of refluxing the concentrated liquid obtained in the filtration step from the membrane separation module to the fermentor through a reflux piping,
(d) a solid matter trapping step of trapping solid matter in the culture liquid by a solid matter removal mechanism disposed on the supply piping, and
(e) a solid matter adding step of adding the trapped solid matter to the culture liquid in the fermentor.

Each step is described below.

2-1. Chemical Production Step (a)

[Microorganism]

In this embodiment, the microorganism is sufficient if it contains a methanogen producing methane, and may be a mixture of a plurality of species. Besides a methanogen, the microorganism may contain an acid former, etc. Thanks to respective actions of a plurality of species, organic matter is decomposed into methane or carbon dioxide. When a chemical other than methane is produced, a microorganism producing a target chemical may be used.

[Feedstock]

The fermentation feedstock (hereinafter, simply referred to as "feedstock") is a substance producing a target chemical through fermentation. The feedstock may be changed according to the microorganism, culture conditions, target chemical, etc. When the chemical sought is methane, the feedstock is a high concentration organic waste such as human waste, livestock excreta, garbage, food waste and activated sludge, or a liquid or solid containing organic matter, such as sludge generated by a treatment of the organic waste and sludge resulting from application of a pretreatment (e.g., pulverization, hydrolysis, solid matter pre-removal) to the organic waste.

The feedstock used for culture contains a component capable of promoting microorganism growth and successfully producing a chemical that is a target fermentation product.

If necessary, organic micronutrients such as amino acids and vitamins may be added.

[Culture Liquid]

The culture liquid contains a feedstock of the chemical and a microorganism being cultured therein and may contain a chemical produced resulting from the culture.

[Culture]

In the continuous fermentation apparatus 100, continuous culture is performed by removing a culture liquid from the fermentor 1 while introducing a feedstock into the fermentor 1.

The introduction of a feedstock is described. In Fig. 1, the feedstock supply pump 12 operates when culture is in progress, and a feedstock is thereby introduced into the fermentor 1 from the feedstock tank 101.

While culture is in progress, introduction of a feedstock may constantly be performed without being stopped, or introduction of a feedstock and stopping thereof may be switched depending on the situation. For example, as described above, the initiation and stopping of the introduction of a feedstock may be performed based on the detection results of the level sensor 81 or may be performed every given time based on the measuring results of a timer (not shown). Note that not only a mode of automatically performing the introduction of a feedstock but also a mode of manually performing the introduction are included in the technical scope of the present invention.

Next, removal of a culture liquid is described. In order to achieve efficient productivity, the concentration of microorganisms in a culture liquid is preferably maintained high to the extent that the environment of the culture liquid does not become inappropriate for proliferation of microorganisms.

In the continuous fermentation apparatus 100, a culture liquid is removed using a circulation system, and continuous culture can thereby be performed while recovering a chemical and keeping the microorganism concentration high. Details of the removal of a culture liquid by using a circulation system are described later.

In addition to the piping 23 connected to the separation membrane module 2, a flow channel for removal may be connected to the fermentor 1, and the removal of a culture liquid may be performed through the flow channel for removal. At this time, not only a liquid portion of the culture liquid but also microorganisms may be removed.

During the culture, a suspension containing fresh microorganisms may be introduced into the fermentor 1. Introduction of microorganisms may be performed either manually or automatically.

In the fermentor, the timings of supply of a feedstock and initiation of the removal of a culture liquid need not necessarily be the same. In addition, the supply of a feedstock and the removal of a culture liquid may be performed successively or intermittently.

For administrative convenience, usually, the continuous culture operation is preferably performed in a single fermentor. However, the number of fermentors is not limited as long as it is a continuous fermentation culture method of generating a product while proliferating microorganisms. For the reason that the fermentor capacity is small, a plurality of fermentors may be used. In this case, high productivity is obtained even when continuous culture is performed by connecting a plurality of fermentors in parallel or in series through piping.

In the continuous fermentation apparatus 100 of Fig. 1, a culture liquid in the fermentor 1 is stirred by means of the stirring device 6, and furthermore, the temperature control unit 5, the pH control unit 7, the level control unit 8, etc. work for maintaining conditions suited for fermentation.

Culture of microorganisms can be performed usually at a pH of 3 or more and 10 or less and at a temperature, which varies depending on the kind of methanogen cultured, of 50°C or more and 60°C or less in high-temperature methane fermentation, 30°C or more and 40°C or less in medium-temperature methane fermentation, and 10°C or more and 20°C or less in low-temperature methane fermentation. The pH of the culture liquid may be adjusted within a predetermined range in the above-described range by using an inorganic or organic acid or an alkaline substance or moreover by using urea, calcium hydroxide, calcium carbonate, ammonia gas, etc. In the continuous fermentation apparatus 100, under the control of the control device 28, the pH is automatically controlled by the pH control unit 7, and the temperature is automatically controlled by the temperature control unit 5.

2-2. Culture Liquid Filtration Step (b)

Because of the filtration step, a chemical can be continuously recovered from a culture liquid and at the same time, the culture can be continued.

In the filtration step, a culture liquid is supplied to the membrane separation module. Specifically, in Fig. 1, a culture liquid is removed from the fermentor 1 by the circulation pump 24 and supplied to the membrane separation module 2 through the piping 23 (supply piping). The culture liquid is separated into a concentrated liquid and a filtrate by the membrane separation module 2. The pump 24 in Fig. 1 corresponds to a circulation pump.

As the filtration method, either cross-flow filtration or dead end filtration may be employed. However, in the continuous fermentation operation, the amount of fouling by microorganisms, etc. attached to the membrane is large and therefore, in order to effectively remove the fouling, it is preferable to perform cross-flow filtration. Because cross-flow filtration enables to remove the fouling by making use of shearing force of the culture liquid.

For the circulation flow rate of a culture liquid at the time of performing cross-flow filtration, the lower the flow rate, the more preferred to reduce the circulation pump power cost. However, when the flow rate is low, the rate at which solid matter flowed into the membrane separation module deposits in the membrane separation module becomes predominant, and clogging of the circulation flow channel progresses. Accordingly, it is desirable to set a circulation flow rate allowing for stable operation while reducing the pump power to the extent possible.

Here, as a result of intensive studies, the present inventors have found a method of setting a circulation flow rate allowing for stable operation while preventing clogging of the circulation flow channel. More specifically, when the linear velocity $v$ [$m^3/m^2/s$] of the membrane surface parallel to the longitudinal direction of the membrane separation module and the smaller one $A$ [m] of major axes of the first and second openings provided on a side surface of the container in the membrane separation module satisfy:

$$v > 0.8(2gA(S-1))^{0.5} \qquad \text{(formula 1)}$$

Stable operation can be achieved. Furthermore, when the membrane surface linear velocity v satisfies:

$$v > 0.8(2.5gA(S-1))^{0.5} \qquad \text{(formula 2)}$$

More stable operation can be achieved.

How these are derived is described blow. As for the equation regarding the deposition rate, the Durand equation shown below is known:

$$V_d = F_d(2gD(S-1))^{0.5}$$

deposition rate: $V_d$ [m/s]
gravity acceleration: g [m/s$^2$]
pipe diameter: D [m]
specific gravity of solid matter: S
$F_d$: empirically determined constant

By reference to the Durand equation, the circulation flow rate allowing for no clogging of the circulation channel in the membrane separation module was experimentally determined. Before the experiment, it is expected that out of the flow channel through which a solid matter-containing liquid (i.e., a culture liquid and a concentrated liquid) passes, deposition of solid matter in the membrane separation module occurs most easily in a narrow portion, namely, between membranes. Accordingly, it is considered that the distance between membranes may be substituted into the pipe diameter D of the Durand equation. Here, the distance between membranes is, specifically, the distance between membrane bundles (the width of the gap between membrane bundles).

However, the present inventors have found that, surprisingly, not the width of the gap between membrane bundles but the smaller one A of major axes of the first and second openings provided on a side surface of the container in the membrane separation module corresponds to the pipe diameter D.

This is considered to be attributable to the fact that the flow of a culture liquid or concentrated liquid passing through the container is bent near the opening provided on a side surface of the container of the membrane separation module. In order to reduce the deposition within the membrane separation module, it is effective to set the circulation flow rate according to the major axis of the opening in a bent part where the flow of a culture liquid or concentrated liquid changes, further set the opening of the solid matter removal mechanism relative to the major axis of the opening in the bent part, and thereby preliminarily remove part of the solid matter.

Above all, in the methane fermentation of producing methane, $F_d$ in the Durand equation is characteristically determined from the average particle diameter of a culture liquid or concentrated liquid flowing through the membrane separation module with a difference from activated sludge-treated wastewater or other fermentation liquids and was experimentally determined to be 0.8.

Based on the foregoing, a large number of experiments were conducted, as a result, it has been found that the circulation flow rate allowing for stable operation in the methane fermentation is in the range satisfying formula (1).

Higher cleaning efficiency can be achieved by further combining scrubbing with the cross-flow filtration. The gas usable for scrubbing includes a compressed gas, etc. supplied by a gas cylinder, blower, compressor, or piping. In addition, a gas produced by metabolism of microorganisms in the fermentor may be collected and supplied.

The execution conductions for scrubbing, i.e., the timing and frequency of executing scrubbing, the time per one scrubbing, etc., are not specifically limited. The execution conditions of scrubbing can be changed depending on various conditions including transmembrane pressure difference, change in the transmembrane pressure difference, pressure in the fermentor, kind of a gas supplied, kind of microorganism cultured, kind of a chemical produced, and kind of feedstock. For example, scrubbing may be performed continuously, performed every time a predetermined time has passed after the completion of previous scrubbing, or performed every time a supply amount of a culture liquid to the membrane separation module 2, that is, a filtration amount or a transmembrane pressure difference, reaches a predetermined value. The continuous fermentation apparatus 100 may be provided with a measuring device such as timer (not shown) to determine the starting and ending times of scrubbing.

A driving force of filtration may be obtained using a syphon making use of a liquid level difference (water head difference) between the fermentor 1 and the membrane separation module 2 or may be obtained using a transmembrane pressure difference that is produced by a circulation pump. In addition, as the driving force of filtration, a suction pump may be disposed on the filtrate side of the membrane separation module 2. In the embodiment of Fig. 1, the filtration pump 14 corresponds to a suction pump. The transmembrane pressure difference can be controlled by the pressure of the suction pump. Furthermore, the transmembrane pressure difference can also be controlled by the pressure of a gas or liquid introduced to the primary side of the membrane separation module 2. A difference between the pressure on the primary side of the membrane separation module 2 and the pressure on the filtrate side is detected as the transmembrane pressure difference and based on this transmembrane pressure difference, control, etc. of the pump can be performed.

In the configuration of Fig. 1, a culture liquid is supplied from the fermentor 1 to the membrane separation module 2 by the circulation pump 24. Depending on the transmembrane pressure difference detected by the pressure difference control unit 9, the operations of the circulation pump 24 and filtration pump 14 are controlled, and the amount of a culture liquid supplied to the membrane separation module 2 is thereby adjusted appropriately.

The filtration can be performed either consecutively or intermittently. In the case of performing the filtration intermittently, filtration can be stopped for a predetermined time (for example, from 0.1 to 10 minutes) every time filtration is continuously conducted, for example, for 5 to 120 minutes. More preferably, filtration is stopped for 0.25 to 3 minutes every time filtration is continued for 5 to 60 minutes. The scrubbing may be performed either during stopping of filtration or during filtration.

2-3. Reflux Step (c)

Microorganisms in a culture liquid do not permeate the separation membrane and therefore, the microorganism concentration is increased in a concentrated liquid having passed through the membrane separation module 2 (a liquid having not permeated the membrane). The concentrated liquid is refluxed to the fermentor 1 through the reflux piping 25, and microorganisms are thereby returned into the fermentor 1. Meanwhile, the filtrate having permeated the separation membrane of the membrane separation module 2 is discharged to the outside of the apparatus though the piping 26.

In this way, the microorganism concentration in the fermentor 1 is maintained high, or a new feedstock is supplied by a portion corresponding to the removal of water containing metabolites, and the fermentation production is continued.

2-4. Solid Matter Trapping Step (Solid Matter Removal Step) (d)

A culture liquid circulated from the fermentor 1 to the membrane separation module 2 passes through the solid matter removal mechanism, thereby letting solid matter larger than the opening be trapped by the removal mechanism, and consequently, the culture liquid supplied to the membrane separation module has a reduced amount of substance to be deposited in the membrane separation module. The solid matter removal mechanism is sufficient if it is provided on a piping providing communication between the fermentor 1 and the membrane separation module 2, and may be disposed either upstream or downstream of the circulation pump.

2-5. Step for Adding Discharged Solid Matter to Culture Liquid (e)

The solid matter trapped by the solid matter removal mechanism in 2-4 above may be directly added into the fermentor as it is or as a suspension or may be added to a culture liquid in the fermentor through at least one route of a piping for refluxing a concentrated liquid from the membrane separation module to the fermentor and a piping connecting the feedstock tank and the fermentor. When the solid matter is added by either method, the solid matter is eventually added to a culture liquid in the fermentor and therefore, the phrase "add the solid matter to a culture liquid in the fermentor" encompasses both of the methods above. The addition may be performed automatically by the movement of the apparatus or may be performed manually.

By such a method, the solid matter is added to a culture liquid without entering the membrane separation module 2 and therefore, is metabolized by microorganisms and effectively utilized as a fermentation feedstock.

The phrase "added directly into the fermentor" means to be added into the fermentor without passing through equipment such as piping and feedstock tank.

Before being added to a culture liquid, the solid matter is discharged from the solid matter removal mechanism. The solid matter may be discharged together with a cleaning liquid by cleaning the solid matter removal mechanism with a cleaning liquid or may be discharged by scraping it out from the solid mater removal mechanism. The solid matter removal mechanism may include a cleaning mechanism or scraping-out mechanism to discharge the solid matter. When the opening is backwashed in a direction opposite to the direction in which a culture liquid permeates, clogging of the removal mechanism can be resolved and at the same time, the cleaning liquid used for backwashing becomes a suspension of solid matter, and thus addition to the culture liquid is easy. These discharging operations may be performed manually but are preferably performed automatically.

As for the timing of discharging, the discharging may be performed when a pressure difference before and after the removal mechanism is detected and reaches a predetermined differential pressure, or may be controlled by a timer to be performed at predetermined time intervals.

2-6. Others

The method for producing a chemical may further include a step of backwashing the separation membrane of the membrane separation module. In the configuration of Fig. 1, a cleaning piping 27 is connected to the secondary side of the membrane separation module 2, so that a cleaning liquid can be charged into the membrane separation module 2 by using the cleaning pump 16.

At the time of conducting the backwashing, the filtration is stopped to prevent the cleaning liquid from flowing into the filtration tank storing a filtrate. That is, the filtration valve 15 is closed, and the filtration pump 14 is stopped. In this state, the cleaning valve 17 opens, the cleaning pump 16 operates, and backwashing is thereby performed.

At the time of stopping the backwashing, the cleaning valve 17 is closed, and the washing pump 16 is stopped. In this state, the filtration valve 15 opens, the filtration pump 14 operates, and filtration is thereby performed.

Control of these can be executed by the control device 28. The continuous fermentation 100 may include a measuring device such as timer (not shown) to determine the starting and ending times of backwashing.

The cleaning liquid used for backwashing includes a liquid having no adverse effect on the fermentation and capable of cleaning the separation membrane, for example, water, a filtrate, a fermentation medium, some components added to the fermentation medium, an aqueous solution of hydrochloric acid, sulfuric acid, nitric acid, sodium hydroxide, calcium hydroxide, or sodium hypochlorite, and a mixed liquid thereof.

3. Chemicals

The chemical obtained by the production method described herein is a substance produced by microorganisms in a culture liquid. The chemicals include, for example, substances produced by a metabolic reaction of microorganism, such as alcohol, organic acid, diamine, amino acid, methane, hydrogen and nucleic acid. The production method described above can also be applied to the production of a substance such as enzyme, antibiotic and recombinant protein.

EXAMPLES

The present invention is more specifically described below by referring to Examples. However, the present invention is not limited to these Examples. The schematic configuration of the continuous fermentation apparatus used in Examples below is as illustrated in Fig. 1 except for the solid matter removal mechanism and the configuration relating to the addition of solid matter discharged from the removal mechanism. I the following examples, lactic acid or methane was produced as a chemical through continuous fermentation.

[A. Method for Measuring D-Lactic Acid Concentration]

The concentration of D-lactic acid contained in a culture liquid was measured by the following method. The concentration was confirmed by sampling 100 $\mu$L of a culture liquid containing D-lactic acid and measuring the lactic acid amount by HPLC method under the conditions shown below.
Column: Shim-Pack SPR-H (produced by Shimadzu)
Mobile phase: 5 mM p-toluenesulfonic acid (flow rate: 0.8 mL/min)
Reaction solution: 5 mM p-toluenesulfonic acid, 20 mM Bis-Tris, 0.1 mM
EDTA·2Na (flow rate: 0.8 mL/min)
Detection method: electrical conductivity
Temperature: 45°C
A calibration curve was created by performing analysis using D-lactic acid having a known concentration as a standard preparation, and plotting the D-lactic acid concentration on the abscissa and a detection peak area on the ordinate.

[B. Method for Measuring Biogas Production Rate]

A gas flow rate sensor (FLOW SIC600, manufactured by SICK AG) was disposed in a flow channel piping connecting a fermentor and a gas tank storing the produced biogas, and the biogas production per hour was monitored. The biogas generation rate was calculated as a gas generation amount (L/d/L) per unit volume of sludge present in the fermenter per day.

[C. Manufacture of Membrane Separation Module]

A pressurized polyvinylidene fluoride hollow fiber module "HFS1020" manufactured by Toray Industries was disassembled and only a portion not fixed with an adhesive was cut out. The polyvinylidene fluoride hollow fiber membrane thus cut out was housed in a case to manufacture a membrane separation module illustrated in Fig. 2.

A container was manufactured by molding a polycarbonate resin, and the major axis of a side opening that is the second opening was changed among examples.

In all of the manufactured membrane separation modules, the capacity was 0.02 L, and the effective filtration area was 200 cm$^2$.

[D. Removal of Solid Matter from Culture Liquid]

A mesh having an aperture size described in each of Examples and Comparative Examples was disposed between the fermentor and the membrane separation module. A liquid was passed through for 10 minutes under the control of a timer, backwashing with sterile water was performed for 1 minute. This operation was repeated. During the backwashing, a culture liquid was fed to the membrane separation module through a mesh of a standby system. The backwashing liquid was added to a culture liquid through a line connected to either of a reflux flow channel from the membrane separation module to the fermentor, or a flow channel for supplying a feedstock to the fermentor.

[E. Preparation of Fermentation Feedstock]

In the case of performing lactic acid fermentation, the fermentation feedstock was obtained according to the following procedure. First, about 25 g of rice straw and 10 g of concentrated sulfuric acid were added and suspended per 990 g of distilled water and subjected to autoclave treatment at 150°C for 30 minutes by using an autoclave (manufactured by Nitto Koatsu Co., Ltd.). After the treatment, a mixed liquid in which the pH was adjusted to near 5 with sodium hydroxide was obtained. Subsequently, 16 g of an aqueous enzyme solution containing a total of 1.6 g of Trichoderma cellulase (produced by Sigma Aldrich Japan) and Novozyme 188 (Aspergillus niger-derived β-glucosidase preparation, produced by Sigma Aldrich Japan) per 1 L of the suspension was prepared, and added to the above-described mixed liquid, followed by stirring and mixing at 50°C for 3 days, and the resultant was served as a fermentation feedstock.

In the case of performing methane fermentation, a simulated garbage was used for the fermentation feedstock. The simulated garbage was composed of 250 g/kg of cabbage, 150 g/kg of potato, 150 g/kg of apple, 200 g/kg of carrot, 30 g/kg of pork, 50 g/kg of cooked white rice, and 50 g/kg of used tea leaves, and this was ground in a mixer and then used as a fermentation feedstock.

[F. Preparation of Genetically Modified Strain Used for Production of D-Lactic Acid Through Continuous Fermentation]

As a microorganism having D-lactic acid producing ability, a yeast *Saccharomyces cerevisiae* having a *Limulus polyphemus*-derived ldh gene introduced into the PDC1 locus, the SED1 locus, and the TDH3 locus was prepared. Specifically, the genetic modification was performed by the method described in WO2010/140602. The strain thus obtained is referred to as SU042 strain. Using the SU042 strain, continuous fermentation of D-lactic acid was performed as described later.

[G. Preparation of Methane Fermentation Sludge Through Continuous Fermentation]

Digested sludge in the urban sewage-treatment plant was used as seed sludge and acclimatized at 35°C while adding the ground simulated garbage. The acclimatization was performed under the conditions of a sludge concentration of 1%, a pH of 7.5 to 8.0, and SRT of 15 days. Using the sludge acclimatized for 30 days, continuous fermentation was performed.

[H. Production of D-Lactic Acid Through Continuous Fermentation]

Unless otherwise specified, the operation conditions of continuous fermentation are as follows.

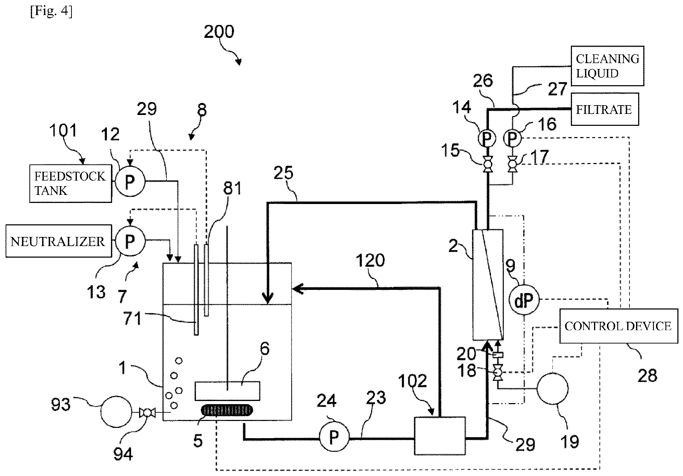

Continuous fermentation of D-lactic acid was conducted using the continuous fermentation apparatus 200 illustrated in Fig. 4. The continuous fermentation apparatus 200 has the same configuration as the continuous fermentation apparatus 100 of Fig. 1 except that it does not include a gas tank 91 and includes an air supply pump 93 and valve 94 for supplying air to the fermentor. Since the lactic acid fermentation is aerobic fermentation, a mechanism for supplying air to the fermentor was thus provided.

In each separation membrane unit, one hollow fiber membrane module manufactured in [C] was used. Common conditions as operation conditions in D-lactic acid continuous fermentation are as follows.

- Volume of fermentation liquid: 1.0 (L)
- Temperature: 32 (°C)
- Fermentor stirring rate: 400 (rpm)
- pH Adjustment: adjusted to pH 4.5 with a 5 N calcium hydroxide suspension
- Circulation pump flow rate: 1.7 L/min
- Filtration rate of membrane separation module: 0.1 $m^3/m^2$/day (fixed)
- The same amount of a fermentation feedstock as the filtration amount was supplied to the fermentor.

[I. Production of Methane Through Continuous Fermentation]

Unless otherwise specified, operation conditions of continuous fermentation are as follows.

Continuous methane fermentation was conducted using the continuous fermentation apparatus 100 illustrated in Fig. 1. In each separation membrane unit, one hollow fiber membrane module manufactured in [C] was used. Common conditions as operation conditions in the methane fermentation are as follows.

- Volume of fermentation liquid: 1.0 (L)
- Temperature: 35 (°C)
- Fermentor stirring rate: 100 (rpm)
- pH Adjustment: none
- Filtration rate of membrane separation module: 0.1 $m^3/m^2$/day (fixed)
- The same amount of a fermentation feedstock as the filtration amount was supplied to the fermentor.
- Circulation pump flow rate: The circulation pump flow rate was appropriately changed to give the target membrane surface linear velocity. The membrane surface linear velocity is calculated by dividing the circulation flow rate [$m^3$/s] of a culture liquid or concentrated liquid by the cross-sectional area [$m^2$] of a hollow fiber membrane present in the cross-section of the flow channel through which a liquid circulates.

[J. Measurement of Average Particle Diameter]

The average particle diameter of a culture liquid or concentrated liquid flowing through the membrane separation module was measured using a laser diffraction particle size distribution measuring method (LA-920, Horiba, Ltd.). Distilled water was used for dispersion medium. The particle diameter was expressed using a volume-based distribution.

[K. Measurement of Specific Gravity]

The specific gravity of a culture liquid or concentrated liquid flowing through the membrane separation module was measured by a sludge specific gravity measurement standard method that is a general sludge test method, in conformity with the "sewage testing method". A specific gravity bottle of a [g] was filled with water, and the obtained weight is denoted by b [g]. Similarly, a specific gravity bottle was filled with a culture liquid or concentrated liquid, and the obtained weight is denoted by c [g]. At this time, the specific gravity of a culture liquid or concentrated liquid is calculated using (c-a)/(b-a).

(Comparative Example 1)

Using a membrane separation module in which the aperture size of the removal mechanism is 2 mm and the major axis of the opening is 5 mm, lactic acid fermentation was performed by letting a liquid discharged from the removal mechanism be added to the fermentor. The results are shown in Table 2. A flow channel of the membrane separation module was clogged by solids in 2 hours after the initiation of circulation to the membrane separation module, and the filtration could not be continued.

(Comparative Example 2)

Using a membrane separation module in which the aperture size of the removal mechanism is 5 mm and the major axis of the opening is 10 mm, lactic acid fermentation was performed by letting a liquid discharged from the removal mechanism be added to the fermentor. A flow channel of the membrane separation module was clogged by solids in 0.5 hours after the initiation of circulation to the membrane separation module, and the filtration could not be continued.

(Comparative Example 3)

Using a membrane separation module in which the aperture size of the removal mechanism is 4 mm and the major axis of the opening is 10 mm, lactic acid fermentation was performed by letting a liquid discharged from the removal mechanism be added to the fermentor. A flow channel of the membrane separation module was clogged by solids in 0.5 hours after the initiation of circulation to the membrane separation module, and the filtration could not be continued.

(Comparative Example 4)

Using a membrane separation module in which the aperture size of the removal mechanism is 10 mm and the major axis of the opening is 15 mm, lactic acid fermentation was performed by letting a liquid discharged from the removal mechanism be added to the fermentor. A flow channel of the membrane separation module was clogged by solids in 10 minutes after the initiation of circulation to the membrane separation module, and the filtration could not be continued.

(Comparative Example 5)

Using a membrane separation module in which the aperture size of the removal mechanism is 20 mm and the major axis of the opening is 50 mm, lactic acid fermentation was performed by letting a liquid discharged from the removal mechanism be added to the fermentor. A flow channel of the membrane separation module was clogged by solids in 10 minutes after the initiation of circulation to the membrane separation module, and the filtration could not be continued.

(Comparative Example 6)

Using a membrane separation module in which the aperture size of the removal mechanism is 30 mm and the major axis of the opening is 72 mm, lactic acid fermentation was performed by letting a liquid discharged from the removal mechanism be added to the fermentor. A flow channel of the membrane separation module was clogged by solids in 10 minutes after the initiation of circulation to the membrane separation module, and the filtration could not be continued.

(Reference Example 1)

Using a membrane separation module in which the aperture size of the removal mechanism is 1 mm and the major axis of the opening is 10 mm, lactic acid fermentation was performed without letting a liquid discharged from the removal mechanism be refluxed and without letting it be added to the fermentor. Stable filtration could be performed with a transmembrane pressure difference of 30 kPa or less for 250 hours, but the average lactic acid accumulated concentration from 100 hours after the initiation of filtration until 150 hours was 3.7 g/L.

(Reference Example 2)

Using a membrane separation module in which the aperture size of the removal mechanism is 0.1 mm and the major axis of the opening is 5 mm, lactic acid fermentation was performed by letting a liquid discharged from the removal mechanism be added to the fermentor. The results are shown in Table 1. Stable filtration could be performed with a transmembrane pressure difference of 30 kPa or less for 250 hours, and the average lactic acid accumulated concentration from 100 hours after the initiation of filtration until 150 hours was 4.2 g/L. In addition, the lactic acid accumulated concentration could be maintained high, compared with Reference Example 1.

(Reference Example 3)

Using a membrane separation module in which the aperture size of the removal mechanism is 1 mm and the major axis of the opening is 5 mm, lactic acid fermentation was performed by letting a liquid discharged from the removal mechanism be added to the fermentor. Stable filtration could be performed with a transmembrane pressure difference of 30 kPa or less for 250 hours, and the average lactic acid accumulated concentration from 100 hours after the initiation of filtration until 150 hours was 4.0 g/L. The lactic acid accumulated concentration could be maintained high, compared with Reference Example 1.

(Reference Example 4)

Using a membrane separation module in which the aperture size of the removal mechanism is 1 mm and the major axis of the opening is 10 mm, lactic acid fermentation was performed by letting a liquid discharged from the removal mechanism be added to the fermentor. Stable filtration could be performed with a transmembrane pressure difference of 30 kPa or less for 250 hours, and the average lactic acid accumulated concentration from 100 hours after the initiation of filtration until 150 hours was 4.1 g/L. The lactic acid accumulated concentration could be maintained high, compared with Reference Example 1.

(Reference Example 5)

Using a membrane separation module in which the aperture size of the removal mechanism is 3 mm and the major axis of the opening is 15 mm, lactic acid fermentation was performed by letting a liquid discharged from the removal mechanism be added to the fermentor. Stable filtration could be performed with a transmembrane pressure difference of 30 kPa or less for 250 hours, and the average lactic acid accumulated concentration from 100 hours after the initiation of filtration until 150 hours was 4.1 g/L. The lactic acid accumulated concentration could be maintained high, compared with Reference Example 1.

(Reference Example 6)

Using a membrane separation module in which the aperture size of the removal mechanism is 10 mm and the major axis of the opening is 30 mm, lactic acid fermentation was performed by letting a liquid discharged from the removal mechanism be added to the fermentor. Stable filtration could be performed with a transmembrane pressure difference of 30 kPa or less for 250 hours, and the average lactic acid accumulated concentration from 100 hours after the initiation of filtration until 150 hours was 4.0 g/L. The lactic acid accumulated concentration could be maintained high, compared with Reference Example 1.

(Reference Example 7)

Using a membrane separation module in which the aperture size of the removal mechanism is 10 mm and the major axis of the opening is 50 mm, lactic acid fermentation was performed by letting a liquid discharged from the removal mechanism be added to the fermentor. Stable filtration could be performed with a transmembrane pressure difference of 30 kPa or less for 250 hours, and the average lactic acid accumulated concentration from 100 hours after the initiation of filtration until 150 hours was 4.1 g/L. The lactic acid accumulated concentration could be maintained high, compared with Reference Example 1.

(Reference Example 8)

Using a membrane separation module in which the aperture size of the removal mechanism is 1 mm and the major axis of the opening is 10 mm, lactic acid fermentation was performed by letting a liquid discharged from the removal mechanism be added to a reflux piping from the membrane separation module to the fermentor. Stable filtration could be performed with a transmembrane pressure difference of 30 kPa or less for 250 hours, and the average lactic acid accumulated concentration from 100 hours after the initiation of filtration until 150 hours was 4.1 g/L. The lactic acid accumulated concentration could be maintained high, compared with Reference Example 1.

(Reference Example 9)

Using a membrane separation module in which the aperture size of the removal mechanism is 1 mm and the major axis of the opening is 10 mm, lactic acid fermentation was performed by letting a liquid discharged from the removal mechanism be added to a feedstock supply piping. Stable filtration could be performed with a transmembrane pressure difference of 30 kPa or less for 250 hours, and the average lactic acid accumulated concentration from 100 hours after the initiation of filtration until 150 hours was 4.2 g/L. The lactic acid accumulated concentration could be maintained high, compared with Reference Example 1.

(Reference Example 10)

Using a membrane separation module in which the aperture size of the removal mechanism is 15 mm and the major axis of the opening is 72 mm, lactic acid fermentation was performed by letting a liquid discharged from the removal mechanism be added to a feedstock supply piping. Filtration could be continues for 250 hours, but the transmembrane pressure difference eventually reached 100 kPa.

The average lactic acid accumulated concentration from 100 hours after the initiation of filtration until 150 hours was 4.0 g/L, and the lactic acid accumulated concentration could be maintained high, compared with Reference Example 1.

(Example 1)

The membrane separation module used had a configuration where a first opening allowing a culture liquid to pass therethrough is provided in the transverse direction of the container and a second opening allowing a concentrated liquid to pass therethrough is provided on a side surface of the container.

Using a membrane separation module in which the aperture size of the removal mechanism is 1 mm and the major axis of the second opening is 5 mm, methane fermentation was performed by letting a liquid discharged from the removal mechanism be added to the fermentor. The circulation flow rate of a culture liquid and concentrated liquid in the membrane separation module was set to be 0.05 $m^3/m^2/s$ in terms of the membrane surface linear velocity on a surface of a hollow fiber membrane included in the membrane separation module. The sludge in the fermentor was sampled at 100 hours after the initiation of filtration. The average particle diameter was 0.4 mm and the specific gravity was 1.03.

Stable filtration could be performed with a transmembrane pressure difference of 30 kPa or less for 250 hours, and the average biogas production rate per unit volume of the sludge from 100 hours after the initiation of filtration until 150 hours was 0.8 L/d/L-sludge.

(Example 2)

A membrane separation module having the same configuration as in Example 1 was used. Using a membrane separation module in which the aperture size of the removal mechanism is 1 mm and the major axis of the second opening is 4 mm, methane fermentation was performed by letting a liquid discharged from the removal mechanism be added to the fermentor. The module was operated at a membrane surface linear velocity of 0.05 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.65 L/d/L.

(Example 3)

A membrane separation module having the same structure as in Example 1 was used. Using a membrane separation module in which the aperture size of the removal mechanism is 0.5 mm and the major axis of the second opening is 4 mm, methane fermentation was performed by letting a liquid discharged from the removal mechanism be added to the fermentor. The module was operated at a membrane surface linear velocity of 0.05 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.7 L/d/L.

(Example 4)

A membrane separation module having the same configuration as in Example 1 was used. Using a membrane separation module in which the aperture size of the removal mechanism is 2 mm and the major axis of the second opening is 8 mm, methane fermentation was performed by letting a liquid discharged from the removal mechanism be added to the fermentor. The module was operated at a membrane surface linear velocity of 0.08 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.9 L/d/L.

(Example 5)

A membrane separation module having the same configuration as in Example 1 was used. Using a membrane separation module in which the aperture size of the removal mechanism is 10 mm and the major axis of the second opening is 50 mm, methane fermentation was performed by letting a liquid discharged from the removal mechanism be added to the fermentor. The module was operated at a membrane surface linear velocity of 0.1 $m^3/m^2/s$. The average particle diameter and specific gravity of the sludge at 100 hours after the initiation of filtration were the same as in Example 1. Stable filtration could be performed with a transmembrane pressure difference of 30 kPa or less for 150 hours, but thereafter, the transmembrane pressure difference reached 50 kPa until 250 hours. The average biogas production rate from 100 hours after the initiation of filtration until 150 hours was 0.7 L/d/L.

(Example 6)

A membrane separation module having the same configuration as in Example 1 was used. Using a membrane separation module in which the aperture size of the removal mechanism is 10 mm and the major axis of the second opening is 50 mm, methane fermentation was performed by letting a liquid discharged from the removal mechanism be added to the fermentor. The module was operated at a membrane surface linear velocity of 0.2 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.85 L/d/L.

(Example 7)

A membrane separation module having the same configuration as in Example 1 was used. Using a membrane separation module in which the aperture size of the removal mechanism is 0.1 mm and the major axis of the second opening is 1 mm, methane fermentation was performed by letting a liquid discharged from the removal mechanism be added to the fermentor. The module was operated at a membrane surface linear velocity of 0.03 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.8 L/d/L.

(Example 8)

A membrane separation module having the same configuration as in Example 1 was used. Using a membrane separation module in which the aperture size of the removal mechanism is 12 mm and the major axis of the second opening is 50 mm, methane fermentation was performed by letting a liquid discharged from the removal mechanism be added to the fermentor. The module was operated at a membrane surface linear velocity of 0.14 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.65 L/d/L.

(Example 9)

A membrane separation module having the same configuration as in Example 1 was used. Using a membrane separation module in which the aperture size of the removal mechanism is 1 mm and the major axis of the second opening is 4 mm, methane fermentation was performed by letting a liquid discharged from the removal mechanism be added to the fermentor. The module was operated at a membrane surface linear velocity of 0.03 $m^3/m^2/s$. The average particle diameter and specific gravity of the sludge at 100 hours after the initiation of filtration were the same as in Example 1. Stable filtration could be performed with a transmembrane pressure difference of 30 kPa or less for 150 hours, but thereafter, the transmembrane pressure difference reached 40 kPa until 250 hours. The average biogas production rate from 100 hours after the initiation of filtration until 150 hours was 0.5 L/d/L.

(Example 10)

A membrane separation module having the same configuration as in Example 1 was used. Using a membrane separation module in which the aperture size of the removal mechanism is 2 mm and the major axis of the second opening is 8 mm, methane fermentation was performed by letting a liquid discharged from the removal mechanism be added to the fermentor. The module was operated at a membrane surface linear velocity of 0.04 $m^3/m^2/s$. The average particle diameter and specific gravity of the sludge at 100 hours after the initiation of filtration were the same as in Example 1. Stable filtration could be performed with a transmembrane pressure difference of 30 kPa or less for 150 hours, but thereafter, the transmembrane pressure difference reached 50 kPa until 250 hours. The average biogas production rate from 100 hours after the initiation of filtration until 150 hours was 0.55 L/d/L.

(Example 11)

A membrane separation module having the same configuration as in Example 1 was used. Using a membrane separation module in which the aperture size of the removal mechanism is 2 mm and the major axis of the second opening is 10 mm, methane fermentation was performed by letting a liquid discharged from the removal mechanism be added to the fermentor. The module was operated at a membrane surface linear velocity of 0.08 $m^3/m^2/s$. The average

particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.8 L/d/L.

(Example 12)

A membrane separation module having the same configuration as in Example 1 was used. Using a membrane separation module in which the aperture size of the removal mechanism is 2.5 mm and the major axis of the second opening is 12 mm, methane fermentation was performed by letting a liquid discharged from the removal mechanism be added to the fermentor. The module was operated at a membrane surface linear velocity of 0.06 $m^3/m^2/s$. The average particle diameter and specific gravity of the sludge at 100 hours after the initiation of filtration were the same as in Example 1. Stable filtration could be performed with a transmembrane pressure difference of 30 kPa or less for 150 hours, but thereafter, the transmembrane pressure difference reached 45 kPa until 250 hours. The average biogas production rate from 100 hours after the initiation of filtration until 150 hours was 0.5 L/d/L.

(Example 13)

A membrane separation module having the same configuration as in Example 1 was used. Using a membrane separation module in which the aperture size of the removal mechanism is 2.5 mm and the major axis of the second opening is 15 mm, methane fermentation was performed by letting a liquid discharged from the removal mechanism be added to the fermentor. The module was operated at a membrane surface linear velocity of 0.09 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.7 L/d/L.

(Example 14)

A membrane separation module having the same configuration as in Example 1 was used. Using a membrane separation module in which the aperture size of the removal mechanism is 1 mm and the major axis of the second opening is 5 mm, methane fermentation was performed by letting a liquid discharged from the removal mechanism be not added to any but be discharged. The module was operated at a membrane surface linear velocity of 0.05 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.45 L/d/L.

(Example 15)

A membrane separation module having the same configuration as in Example 1 was used. Using a membrane separation module in which the aperture size of the removal mechanism is 1 mm and the major axis of the second opening is 5 mm, methane fermentation was performed by letting a liquid discharged from the removal mechanism be added through a line connected to a feedstock shared piping. The module was operated at a membrane surface linear velocity of 0.05 $m^3/m^2/s$. The average particle diameter and specific gravity of the sludge at 100 hours after the initiation of filtration were the same as in Example 1. Stable filtration could be performed with a transmembrane pressure difference of 30 kPa or less for 250 hours, and the average biogas production rate from 100 hours after the initiation of filtration until 150 hours was 0.75 L/d/L.

Table 1

| | A. Aperture Size of Removal Mechanism [mm] | B. Length in Longitudinal Direction of Opening of Membrane Separation Mechanism [mm] | C. 1/3 in Longitudinal Direction of Opening [mm] | Size of A Relative to C | Place to Which Liquid Discharged from Removal Mechanism is Refluxed | Average Lactic Acid Accumulated Concentration from 100 Hours until 150 Hours [g/L] | Filtration State |
|---|---|---|---|---|---|---|---|
| Comparative Example 1 | 2.0 | 5.0 | 1.7 | larger | fermentor | - | The flow channel of the membrane separation mechanism was clogged by solid matter and the filtration could not be continued. |
| Comparative Example 2 | 5.0 | 10.0 | 3.3 | larger | fermentor | - | The flow channel of the membrane separation mechanism was clogged by solid matter and the filtration could not be continued. |
| Comparative Example 3 | 4.0 | 10.0 | 3.3 | larger | fermentor | - | The flow channel of the membrane separation mechanism was clogged by solid matter and the filtration could not be continued. |
| Comparative Example 4 | 10.0 | 15.0 | 5.0 | larger | fermentor | - | The flow channel of the membrane separation mechanism was clogged by solid matter and the filtration could not be continued. |
| Comparative Example 5 | 20.0 | 50.0 | 17 | larger | fermentor | - | The flow channel of the membrane separation mechanism was clogged by solid matter and the filtration could not be continued. |
| Comparative Example 6 | 30.0 | 72.0 | 24 | larger | fermentor | - | The flow channel of the membrane separation mechanism was clogged by solid matter and the filtration could not be continued. |

Table 2

| | A. Aperture Size of Removal Mechanism [mm] | B. Length in Longitudinal Direction of Opening of Membrane Separation Mechanism [mm] | C. 1/3 in Longitudinal Direction of Opening [mm] | Size of A Relative to C | Place to Which Liquid Discharged from Removal Mechanism is Refluxed | Average Lactic Acid Accumulated Concentration from 100 Hours until 150 Hours [g/L] | Filtration State |
|---|---|---|---|---|---|---|---|
| Reference Example 1 | 1.0 | 10.0 | 3.3 | smaller | not refluxed | 3.7 | Stable filtration could be performed with a transmembrane pressure difference of 30 kPa or less for 250 hours. |
| Reference Example 2 | 0.1 | 5.0 | 1.7 | smaller | fermentor | 4.2 | Stable filtration could be performed with a transmembrane pressure difference of 30 kPa or less for 250 hours. |
| Reference Example 3 | 1.0 | 5.0 | 1.7 | smaller | fermentor | 4.0 | Stable filtration could be performed with a transmembrane pressure difference of 30 kPa or less for 250 hours. |
| Reference Example 4 | 1.0 | 10.0 | 3.3 | smaller | fermentor | 4.1 | Stable filtration could be performed with a transmembrane pressure difference of 30 kPa or less for 250 hours. |
| Reference Example 5 | 3.0 | 15.0 | 5.0 | smaller | fermentor | 4.1 | Stable filtration could be performed with a transmembrane pressure difference of 30 kPa or less for 250 hours. |
| Reference Example 6 | 10.0 | 30.0 | 10 | smaller | fermentor | 4.0 | Stable filtration could be performed with a transmembrane pressure difference of 30 kPa or less for 250 hours. |

| | A. Aperture Size of Removal Mechanism [mm] | B. Length in Longitudinal Direction of Opening of Membrane Separation Mechanism [mm] | C. 1/3 in Longitudinal Direction of Opening [mm] | Size of A Relative to C | Place to Which Liquid Discharged from Removal Mechanism is Refluxed | Average Lactic Acid Accumulated Concentration from 100 Hours until 150 Hours [g/L] | Filtration State |
|---|---|---|---|---|---|---|---|
| Reference Example 7 | 10.0 | 50.0 | 17 | smaller | fermentor | 4.1 | Stable filtration could be performed with a transmembrane pressure difference of 30 kPa or less for 250 hours. |
| Reference Example 8 | 1.0 | 10.0 | 3.3 | smaller | reflux piping from membrane separation mechanism to fermentor | 4.1 | Stable filtration could be performed with a transmembrane pressure difference of 30 kPa or less for 250 hours. |
| Reference Example 9 | 1.0 | 10.0 | 3.3 | smaller | feedstock supply piping to fermentor | 4.2 | Stable filtration could be performed with a transmembrane pressure difference of 30 kPa or less for 250 hours. |
| Reference Example 10 | 15.0 | 72.0 | 24 | smaller | fermentor | 4.0 | Filtration could be continued, but the transmembrane pressure difference increased to 100 kPa. |

Table 3

| | X. Aperture Size of Removal Mechanism [mm] | Y. Major Axis A of Opening of Membrane Separation Mechanism [mm] | Z. 1/3 of Major Axis of Opening [mm] | Size of X Relative to Z | Place to Which Liquid Discharged from Removal Mechanism is Refluxed | Surface Linear Velocity of Culture Liquid [m$^3$/m$^2$/s] | Membrane Biogas Production Rate [L/d/L-sludge] | Filtration State |
|---|---|---|---|---|---|---|---|---|
| Example 1 | 1.0 | 5.0 | 1.7 | smaller | fermentor | 0.05 | 0.80 | Stable with a transmembrane pressure difference of 30 kPa or ess until 250 hours. |
| Example 2 | 1.0 | 4.0 | 1.3 | smaller | fermentor | 0.05 | 0.65 | Stable with a transmembrane pressure difference of 30 kPa or ess until 250 hours. |
| Example 3 | 0.5 | 4.0 | 1.3 | smaller | fermentor | 0.05 | 0.70 | Stable with a transmembrane pressure difference of 30 kPa or ess until 250 hours. |
| Example 4 | 2.0 | 8.0 | 2.7 | smaller | fermentor | 0.08 | 0.90 | Stable with a transmembrane pressure difference of 30 kPa or less until 250 hours. |
| Example 5 | 10.0 | 50.0 | 16.7 | smaller | fermentor | 0.1 | 0.70 | Stable until 150 hours and thereafter, the transmembrane pressure difference was increased to 50 kPa until 250 hours. |
| Example 6 | 10.0 | 50.0 | 16.7 | smaller | fermentor | 0.2 | 0.85 | Stable with a transmembrane pressure difference of 30 kPa or ess until 250 hours. |
| Example 7 | 0.1 | 1.0 | 0.3 | smaller | fermentor | 0.03 | 0.80 | Stable with a transmembrane pressure difference of 30 kPa or ess until 250 hours. |
| Example 8 | 12.0 | 50.0 | 16.7 | smaller | fermentor | 0.14 | 0.65 | Stable with a transmembrane pressure difference of 30 kPa or ess until 250 hours. |

| | X. Aperture Size of Removal Mechanism [mm] | Y. Major Axis A of Opening of Membrane Separation Mechanism [mm] | Z. 1/3 of Major Axis of Opening [mm] | Size of X Relative to Z | Place to Which Liquid Discharged from Removal Mechanism is Refluxed | Membrane Surface Linear Velocity of Culture Liquid [m³/m²/s] | Biogas Production Rate [L/d/L-sludge] | Filtration State |
|---|---|---|---|---|---|---|---|---|
| Example 9 | 1.0 | 4.0 | 1.3 | smaller | fermentor | 0.03 | 0.50 | Stable until 150 hours and thereafter, the transmembrane pressure difference was increased to 40 kPa until 250 hours. |
| Example 10 | 2.0 | 8.0 | 2.7 | smaller | fermentor | 0.04 | 0.55 | Stable until 150 hours and thereafter, the transmembrane pressure difference was increased to 50 kPa until 250 hours. |
| Example 11 | 2.0 | 10.0 | 3.3 | smaller | fermentor | 0.08 | 0.80 | Stable with a transmembrane pressure difference of 30 kPa or ess until 250 hours. |
| Example 12 | 2.5 | 12.0 | 4.0 | smaller | fermentor | 0.06 | 0.50 | Stable until 150 hours and thereafter, the transmembrane pressure difference was increased to 45 kPa until 250 hours. |
| Example 13 | 2.5 | 15.0 | 5.0 | smaller | fermentor | 0.09 | 0.70 | Stable with a transmembrane pressure difference of 30 kPa or less until 250 hours. |
| Example 14 | 1.0 | 5.0 | 1.7 | smaller | discharged | 0.05 | 0.45 | Stable with a transmembrane pressure difference of 30 kPa or ess until 250 hours. |
| Example 15 | 1.0 | 5.0 | 1.7 | smaller | feedstock supply piping | 0.05 | 0.75 | Stable with a transmembrane pressure difference of 30 kPa or ess until 250 hours. |

| | X. Aperture Size of Removal Mechanism [mm] | Y. Major Axis A of Opening of Membrane Separation Mechanism [mm] | Z. 1/3 of Major Axis of Opening [mm] | Size of X Relative to Z | Place to Which Liquid Discharged from Removal Mechanism is Refluxed | Membrane Surface Linear Velocity of Culture Liquid [m$^3$/m$^2$/s] | Biogas Production Rate [L/d/L-sludge] | Filtration State |
|---|---|---|---|---|---|---|---|---|
| Comparative Example 7 | 2.0 | 5.0 | 1.7 | larger | fermentor | - | - | The flow channel of the membrane separation mechanism was clogged by solid matter and the filtration could not be continued. |

**EP 3 647 428 A1**

INDUSTRIAL APPLICABILITY

The present invention can simultaneously realize the long-term stability of separation membrane operation and the enhancement of fermentation results and therefore, be widely utilized for microbial fermentation applications, and it becomes possible to stably produce a chemical as a product at low cost.

While the invention has been described in detail and with reference to specific embodiments thereof, it will be apparent to one skilled in the art that various changes and modifications can be made therein without departing from the spirit and scope thereof.

The present application is based on Japanese Patent Application filed on June 30, 2017 (Japanese Patent Application No. 2017-128606), the contents of which are incorporated herein by reference.

REFERENCE SIGNS LIST

1 Fermentor
2 Membrane separation module
31 Lower potting
311 Hole of lower potting
32 Upper potting
5 Temperature control unit
6 Stirring device
7 pH Control unit
8 Level control unit
9 Pressure difference control unit
12 Feedstock supply pump
13 Neutralizer supply pump
14 Filtration pump
15 Filtration valve
16 Cleaning pump
17 Cleaning valve
18 Gas supply control valve
19 Gas supply device
23 Supply piping providing communication between the fermentor 1 and the primary side of the membrane separation module 2
24 Circulation pump
25 Reflux piping for returning a concentrated liquid having not permeated the separation membrane of the membrane separation module 2 to the fermentor 1
26 Piping connected to the membrane separation module 2 for discharging a filtrate to the outside of the apparatus
27 Piping for connecting a cleaning liquid tank and the secondary side of the membrane separation module 2
28 Control device
29 Feedstock supply piping
71 pH Sensor
81 Level sensor
90 Discharge line for gas
91 Gas tank
100 Continuous fermentation apparatus
101 Feedstock tank
102 Solid matter removal mechanism
120 Solid matter addition piping
103 Container
104 Separation membrane
105 Bottom opening (first opening)
106 Side opening (second opening)
111 Flow-regulating member
112 Flow-regulating hole

**Claims**

1.  An apparatus for producing a chemical, comprising:

    a fermentor for housing a feedstock and a culture liquid containing a microorganism capable of producing methane through fermentation from the feedstock,
    an external pressure-type membrane separation module for separating the microorganism from the culture liquid to obtain a filtrate and a concentrated liquid,
    a supply piping for supplying the culture liquid in the fermentor to the membrane separation module,
    a reflux piping for refluxing the concentrated liquid from the membrane separation module into the fermentor, and
    a solid matter removal mechanism disposed on the supply piping for trapping a part of the solid matter in the culture liquid, wherein
    the membrane separation module comprises a container, a separation membrane housed in the container, a first opening allowing the culture liquid flowing into the container to pass therethrough, and a second opening allowing the concentrated liquid flowing out of the container to pass therethrough, the openings being provided in the container,
    the first opening and the second opening are arranged apart from each other in the longitudinal direction of the container,
    the first opening and/or the second opening is provided on a side surface of the container, and
    an aperture size of the solid matter removal mechanism is 1/3 or less of a smaller one of major axes of the first and second openings provided on the side surface of the container.

2.  The apparatus for producing a chemical according to claim 1, wherein the aperture size of the solid matter removal mechanism is 0.1 mm or more.

3.  The apparatus for producing a chemical according to claim 1 or 2, wherein the aperture size of the solid matter removal mechanism is 10 mm or less.

4.  The apparatus for producing a chemical according to claim 3, wherein the aperture size of the solid matter removal mechanism is 2 mm or less.

5.  The apparatus for producing a chemical according to any one of claims 1 to 4, further comprising a solid matter addition piping through which the solid matter trapped by the solid matter removal mechanism is added to the culture liquid in the fermentor.

6.  A method for producing a chemical by using the apparatus for producing a chemical according to any one of claims 1 to 5, comprising:
    a step of flowing a culture liquid into a membrane separation module such that the membrane surface linear velocity $v$ [$m^3/m^2/s$] of the culture liquid in the membrane separation module in the longitudinal direction of the container and the smaller one $A$ [m] of major axes of the first and second openings provided on the side surface of the container satisfy $v>0.8(2gA(S-1))^{0.5}$.

7.  A method for producing a chemical by using the apparatus for producing a chemical according to any one of claims 1 to 5, comprising:
    a step of directly adding the solid matter trapped by the solid matter removal mechanism to the culture liquid in the fermentor.

8.  A method for producing a chemical by using the apparatus for producing a chemical according to any one of claims 1 to 5, comprising:
    a step of adding the solid matter trapped by the solid matter removal mechanism to the culture liquid in the fermentor through the reflux piping.

9.  A method for producing a chemical by using the apparatus for producing a chemical according to any one of claims 1 to 5 which further includes a feedstock supply piping connected to the fermentor, comprising:

    a step of adding a fermentation feedstock to the fermentor through the feedstock supply piping, and
    a step of adding the solid matter trapped by the solid matter removal mechanism to the culture liquid in the fermentor through the feedstock supply piping.

[Fig. 1]

[Fig. 2]

[Fig. 3]

[Fig. 4]

## INTERNATIONAL SEARCH REPORT

| International application No. |
| --- |
| PCT/JP2018/024978 |

**A. CLASSIFICATION OF SUBJECT MATTER**
Int.Cl.    C12P1/00(2006.01)i,    B01D61/14(2006.01)i,    C12M1/12(2006.01)i,
C12P7/56(2006.01)n
According to International Patent Classification (IPC) or to both national classification and IPC

**B. FIELDS SEARCHED**

Minimum documentation searched (classification system followed by classification symbols)
Int.Cl. C12P1/00, B01D61/14, C12M1/12, C12P7/56

| Documentation searched other than minimum documentation to the extent that such documents are included in the fields searched | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
CAplus/MEDLINE/EMBASE/BIOSIS (STN), JSTPlus/JMEDPlus/JST7580 (JDreamIII),
PubMed, Japio-GPG/FX

**C. DOCUMENTS CONSIDERED TO BE RELEVANT**

| Category* | Citation of document, with indication, where appropriate, of the relevant passages | Relevant to claim No. |
| --- | --- | --- |
| A | JP 2012-179018 A (TORAY INDUSTRIES, INC.) 20 September 2012, claim 1 (Family: none) | 1–9 |
| A | WO 2007/097260 A1 (TORAY INDUSTRIES, INC.) 30 August 2007, claim 23 & US 2009/0269812 A1, claim 23 & JP 2007-252367 A & JP 2008-048721 A & EP 1988170 A1 | 1–9 |
| A | JP 2006-326534 A (KOBELCO ECO-SOLUTIONS CO., LTD.) 07 December 2006, claim 7 (Family: none) | 1–9 |
| A | JP 2002-205090 A (TOSHIBA CORP.) 23 July 2002, claim 1 (Family: none) | 1–9 |

☐ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 18 September 2018 (18.09.2018) | 02 October 2018 (02.10.2018) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 07097260 A
- JP 2006326534 A
- WO 2010140602 A
- JP 2017128606 A

**Non-patent literature cited in the description**

- Kagaku Binran Kisohen Kaitei 3rd Edition. The Chemical Society of Japan, 1984, II-82